# EUROPÄISCHE PATENTSCHRIFT

(11) **EP 3 454 769 B1**
(45) Veröffentlichungstag und Bekanntmachung des Hinweises auf die Patenterteilung: **10.06.2020**
(21) Anmeldenummer: 17733349.9
(22) Anmeldetag: 09.05.2017
(51) Int. Cl.: A61B 34/00, B25J 9/10, B25J 19/00, A61B 34/30, A61B 17/29

(54) **INSTRUMENT, INSBESONDERE MEDIZINISCH-ENDOSKOPISCHES INSTRUMENT**
INSTRUMENT, IN PARTICULAR MEDICAL ENDOSOSCOPIC INSTRUMENT
INSTRUMENT, EN PARTICULIER INSTRUMENT D'ENDOSCOPIE MÉDICALE

(30) Priorität: 12.05.2016 DE 102016208218
(43) Veröffentlichungstag der Anmeldung: 20.03.2019
(73) Patentinhaber: Richard Wolf GmbH, 75438 Knittlingen (DE)
(72) Erfinder: WEHRHEIM, Frank, 75015 Bretten (DE)
(74) Vertreter: Patentanwälte Vollmann Hemmer Lindfeld Partnerschaft mbB
(86) Internationale Anmeldenummer: PCT/DE2017/200040
(87) Internationale Veröffentlichungsnummer: WO 2017/194064

(56) Entgegenhaltungen:
- EP-A1- 3 106 101
- DE-A1-102014 206 930
- DE-A1-102014 219 195
- US-A1- 2014 117 686

## Beschreibung

Die Erfindung betrifft ein Instrument und insbesondere ein medizinisch-endoskopisches Instrument mit den im Oberbegriff von Anspruch 1 angegebenen Merkmalen.

Im Bereich der minimal-Invasiven Chirurgie werden Instrumente eingesetzt, die an dem distalen Ende eines Schafts einen gegenüber dem Schaft abwinkelbaren Instrumentenkopf mit einem daran angeordneten Werkzeug aufweisen. Diese Instrumente werden durch einen natürlichen oder künstlich geschaffenen Zufuhrkanal in das Körperinnere des zu behandelnden Patienten eingeführt, so dass das Werkzeug in unmittelbarer Nähe des Behandlungsgegenstandes angeordnet werden kann, während sich proximalseitig des Schafts angeordnete Einrichtungen zur Steuerung des Werkzeugs für den Operateur gut zugänglich außerhalb des Patienten befinden. Instrumente dieser Art finden darüber hinaus auch auf anderen Gebieten, beispielsweise bei Technoskopen zum Einsatz in Hohlräumen technischer Objekte, Verwendung.

Den Ausgangspunkt der Erfindung bilden solche Instrumente, bei denen der Instrumentenkopf am distalen Ende des Schafts über ein Doppelgelenk mit zwei in Längsrichtung des Instruments voneinander beabstandeten Gelenkachsen angelenkt ist, wobei der Instrumentenkopf über einen an seinem proximalen Ende ausgebildeten Wälzkörper auf einem an dem distalen Ende des Schafts ausgebildeten Wälzkörper abwälzt. Zur Bewegungssteuerung des Instrumentenkopfs werden üblicherweise Seilzüge verwendet, die mit dem Instrumentenkopf antagonistisch wirkend bewegungsgekoppelt sind. Derartige Instrumente sind beispielsweise aus DE 102014219195 A1 und DE 102014206930 A1 bekannt.

Des Weiteren ist aus der US 2014/0117686 A1, insbesondere Abbildungen 5 und 6, eine Roboterhand mit quer zur Mittelachse eines Befestigungsabschnitts bewegbaren Seilspannrollen bekannt.

Insbesondere bei Instrumenten, bei denen die Krafteinleitung zum Abwinkein des Instrumentenkopfs über die Seilzüge direkt an dem Instrumentenkopf also distalseitig des Doppelgelenks erfolgt, stellen außenseitig auf das an dem Instrumentenkopf angeordnete Werkzeug wirkende Störkräfte ein Problem dar, da sie zu einer unerwünschten Änderung der Ausrichtung des Instrumentenkopfs relativ zu dem Schaft führen können. Diese Problematik tritt typischerweise auch auf, wenn Störkräfte direkt auf den Instrumentenkopf wirken, so dass nachfolgend immer dann, wenn von auf das Werkzeug wirkenden Störkräften die Rede ist, hierunter auch direkt auf den Instrumentenkopf wirkende Störkräfte zu verstehen sind.

Vor dem Hintergrund dieser Problematik besteht die Aufgabe der Erfindung darin, ein Instrument der eingangs beschriebenen Art zu schaffen, bei dem die nachteiligen Auswirkungen von außenseitig auf das an dem Instrumentenkopf angeordnete Werkzeug wirkenden Störkräften zumindest in erheblichem Maße verringert sind und günstigstenfalls gar nicht auftreten.

Diese Aufgabe wird durch ein Instrument mit den in Anspruch 1 angegebenen Merkmalen gelöst, wobei sich vorteilhafte Weiterbildungen dieses Instruments aus den Unteransprüchen, der nachfolgenden Beschreibung sowie der Zeichnung ergeben. Gemäß der Erfindung können hierbei die Unteransprüche jeweils für sich aber auch in sinnvoller Kombination miteinander das Instrument nach Anspruch 1 weiter ausgestalten.

Das erfindungsgemäße Instrument ist bevorzugt ein medizinisch-endoskopisches Instrument, es kann sich bei ihm aber auch um ein Technoskop handeln, das in schwer zugänglichen Hohlräumen technischer Objekte eingesetzt wird. Darüber hinaus kann das Instrument als ein manuell betätigbares Instrument ausgebildet sein oder Teil eines robotischen Systems bilden. Unabhängig hiervon weist das Instrument einen länglichen, vorzugsweise gerade und starr ausgebildeten Schaft auf, distalseitig dessen, ein Instrumentenkopf angeordnet ist. Dieser Instrumentenkopf trägt distalseitig ein Werkzeug. Die Art dieses Werkzeugs ist grundsätzlich beliebig, bei dem erfindungsgemäßen Instrument ist allerdings bevorzugt ein Maulwerkzeug mit zwei relativ zueinander verschwenkbaren Maulteilen vorgesehen, wobei die Maulteile von proximalseitig des Schafts mit durch den Schaft geführten Betätigungsmitteln steuerbar sind.

Der Instrumentenkopf ist über ein Doppelgelenk mit zwei in Längsrichtung des Instruments voneinander beabstandeten Gelenkachsen an dem Schaft angelenkt. Hierbei ist unter einem Doppelgelenk ein solches Gelenk zu verstehen, das einen Gelenkteil aufweist, der um eine an dem distalen Ende des Schafts ausgebildete Gelenkachse, die nachfolgend als proximale Gelenkachse bezeichnet wird, schwenkbar ist, wobei der Instrumentenkopf distalseitig an dem Gelenkteil um eine zweite, nachfolgend als distale Gelenkachse bezeichnete Gelenkachse abwinkelbar angelenkt ist. Somit ist der Instrumentenkopf relativ zu dem Schaft derart abwinkelbar, dass sich seine Gesamtabwinkelung relativ zu dem Schaft aus der Summe der Abwinkelung des Gelenkteils relativ zu dem Schaft und seiner Abwinkelung relativ zu dem Gelenkteil ergibt.

Zur Steuerung der Abwinkelung des Instrumentenkopfs ist dieser mit zwei antagonistisch auf den Instrumentenkopf wirkenden Seilzügen bewegungsverbunden. Das heißt, die Bewegungsverbindung der beiden Seilzüge mit dem Instrumentenkopf ist dergestalt, dass einer der Seilzüge als Agonist bei einer Zugbeanspruchung einer Abwinkelung des Instrumentenkopfs in eine erste Richtung hervorruft, wobei der andere Seilzug als Antagonist einen Gegenspieler zu dem ersten Seilzug bildet und bei einer Zugbeanspruchung als Agonist eine Gegenbewegung des Instrumentenkopfs hervorruft. Bei den beiden Seilzügen kann es sich um zwei separate Seile handeln, die jeweils mit dem Instrumentenkopf bewegungsverbunden sind. Daneben besteht auch die Möglichkeit, dass zur Bildung der beiden Seilzüge lediglich ein Seil verwendet wird, welches unter Bildung einer Schlaufe mit dem Instrumentenkopf bewegungsgekoppelt ist, wobei dann die beiden Enden des Seils jeweils einen der Seilzüge bilden. Die Seilzüge sind durch den Schaft geführt und proximalseitig des Schafts mit einer Steuerungseinrichtung wirkungsverbunden. Bei der Steuerungseinrichtung kann es sich, je nach Art des Instruments, um eine Handhabe zur manuellen Betätigung oder um eine Steuerungsschnittstelle eines robotischen Systems handeln.

Im Bereich des distalen Endes des Schafts ist eine Seilführung angeordnet, welche zwei Führungsflächen aufweist, an denen jeweils einer der Seilzüge anliegt. Die Führungsflächen sind in der Seilführung einander in radialer Richtung des Schafts zugewandt und weisen vorzugsweise einen radialen Abstand voneinander auf, welcher geringer als der Abstand der beiden Seilzüge distalseitig der Seilführung ist. Demzufolge stützen sich die an dem Instrumentenkopf festgelegten Seilzüge an den Führungsflächen in radialer Richtung des Schafts nach außen, das heißt in Richtung von einer Mittelachse des Schafts weg, ab, wobei sie gegenüber ihrer Anordnung distalseitig der Seilführung in Richtung der Mittelachse des Schafts in gewissem Maße zusammengeführt sind.

Die maßgebliche Besonderheit der Erfindung besteht darin, dass die Führungsflächen der Seilführung synchron in gleicher Richtung quer zu der Mittelachse des Schafts also in radialer Richtung des Schafts bewegbar sind. Diese Bewegbarkeit der Führungsflächen erweist sich insbesondere dann als vorteilhaft, wenn von außen auf das an dem Instrumentenkopf angeordnete Werkzeug eine Störkraft wirkt, welche bestrebt ist, eine gewünschte Ausrichtung des Instrumentenkopfs relativ zu dem Schaft zu verändern. Um dem entgegenwirken zu können, ist eine Erhöhung der Zugkraft an dem als Agonist wirkenden Seilzug bzw. eine Erhöhung des von dieser Zugkraft auf den Instrumentenkopf ausgeübten Drehmoments erforderlich. Dies wird dadurch bewirkt, dass die Führungsfläche der Seilführung, an welcher der den Agonisten bildenden Seilzug anliegt, soweit in radialer Richtung von der Mittelachse des Schafts weg nach außen bewegt wird, bis sich zwischen den von der Störkraft und den von den auf die beiden Seilzüge wirkenden Kräften auf den Instrumentenkopf ausgeübten Drehmomenten ein Gleichgewichtszustand einstellt, indem sich der Angriffshebel des Agonisten zu einem Momentanpol des Doppelgelenks und hiermit das von den Agonisten verursachte Drehmoment durch die Bewegung des als Agonist wirkenden Seilzugs vergrößert.

Die Seilzüge des erfindungsgemäßen Instruments sind zweckmäßigerweise zwischen ihrer Anbindung an dem Instrumentenkopf und ihrer Anbindung an der proximalseitig des Schafts angeordneten Steuerungseinrichtung vorgespannt. Bei einer auf das Werkzeug bzw. auf den Instrumentenkopf wirkenden Störkraft erhöht sich die Spannung in dem den Agonisten bildenden Seilzug, der sich dadurch längt, während in dem den Antagonisten bildenden Seilzug die Vordehnung abnimmt, wodurch dieser Seilzug seine Spannung vollständig verlieren kann. Dies kann bei einem die beiden Seilzüge bildenden Seil ungünstigstenfalls dazu führen, dass das Seil, welches distalseitig der proximalen Gelenkachse des Doppelgelenks vorzugsweise an weiteren Umlenk- und/oder Führungsrollen geführt ist, von einer dieser Rollen abspringt, wodurch das Instrument unbrauchbar wird. Um dem entgegenzuwirken, ist zweckmäßigerweise auch die Führungsfläche der Seilführung, an welcher der Antagonist anliegt, derart gezielt in radialer Richtung des Schafts so zu bewegen, dass sich die Spannung in dem den Antagonisten bildenden Seilzug wieder erhöht. In diesem Zusammenhang ist die erfindungsgemäße Ausgestaltung vorteilhaft, bei welcher die Führungsflächen des Seilzugs synchron in gleicher Richtung bewegbar sind. Besonders vorteilhaft ist hierbei die gesamte Seilführung zumindest in Richtung quer zur Mittelachse des Schafts bewegbar. Dementsprechend wird bei einer bei abgewinkeltem Instrumentenkopf auf das Werkzeug wirkenden Störkraft bevorzugt nicht nur die Führungsfläche, an welcher der den Agonisten bildende Seilzug anliegt, in radialer Richtung nach außen bewegt, sondern gleichzeitig auch die Führungsfläche, an welcher der den Antagonisten bildende Seilzug anliegt, in die gleiche Richtung wie die andere Führungsfläche bewegt, wobei sich die Spannung in dem den Antagonisten bildenden Seilzug erhöht.

Vorteilhafterweise sind die Führungsflächen aufgrund einer Erhöhung der Kraftwirkung auf den als Agonisten wirkenden Seilzug selbsttätig bewegbar. Eine Erhöhung der Kraftwirkung auf den als Agonist wirkenden Seilzug findet dann statt, wenn auf das Werkzeug eine quer zur Mittelachse des Instrumentenkopfs gerichtete Störkraft wirkt. Da der den Agonisten bildende Seilzug distalseitig der Seilführung insbesondere bei abgewinkeltem Instrumentenkopf einen größeren radialen Abstand von der Mittelachse des Schafts als in der Seilführung hat, wird von dem als Agonist wirkenden Seilzug auf die Führungsfläche der Seilführung, an der er anliegt, eine quer zur Mittelachse des Schafts nach außen gerichtete Kraft ausgeübt. Mit der von einer Störkraft an dem Werkzeug verursachten Erhöhung der Kraftwirkung auf den als Agonist wirkenden Seilzug geht somit auch eine Erhöhung der in Richtung quer zur Mittelachse des Schafts nach außen gerichteten Kraft auf die Führungsfläche für diesen Seilzug einher. Hierdurch wird diese Führungsfläche, die in dem Schaft zumindest in Richtung quer zur Mittelachse des Schafts frei bewegbar angeordnet ist, in dem Schaft radial nach außen bewegt, bis sich ein Kräftegleichgewicht zwischen der Störkraft und den an den beiden Seilzügen wirkenden Kräften eingestellt hat. Die mit dieser Führungsfläche synchron bewegbare andere Führungsfläche, welche in Richtung quer zur Mittelachse des Schafts frei bewegbar ist, wird hierbei in die gleiche Richtung wie die Führungsfläche bewegt, an welcher der den Agonisten bildende Seilzug anliegt.

Die Führungsflächen für die beiden zur Abwinkelung des Instrumentenkopfs vorgesehenen Seilzüge können prinzipiell von beliebigen Führungskörpern gebildet werden. Hinsichtlich der Reibung zwischen den Führungsflächen und den daran anliegenden Seilzügen erweist es sich aber als vorteilhaft, wenn quer zur Mittelachse des Schafts drehbar gelagerte Führungskörper verwendet werden. In diesem Sinne ist erfindungsgemäß bevorzugt vorgesehen, dass die Seilführung zwei in radialer Richtung des Schafts voneinander beabstandete Seilrollen aufweist, welche jeweils eine Führungsfläche für einen Seilzug bilden. Hierbei weisen die Drehachsen der beiden Seilrollen bei einer Normalstellung der Seilführung, in welcher die Seilführung nicht aufgrund einer auf das Werkzeug bzw. auf den Instrumentenkopf wirkenden Störkraft quer zur Mittelachse des Schafts bewegt ist, vorzugsweise jeweils den gleichen Abstand von der Mittelachse des Schafts auf. Des Weiteren weisen die Seilrollen bevorzugt auch einen identischen Durchmesser auf. Die Seilzüge sind zwischen den Seilrollen hindurchgeführt und stützen sich an den einander zugewandten Seiten der Seilrollen ab. Wird einer der Seilzüge zur Steuerung der Abwinkelung des Instrumentenkopfs auf Zug beansprucht, führt dies zu einer Drehbewegung der entsprechenden Seilrolle, an der dieser Seilzug anliegt. Insofern besteht zwischen dem Seilzug und der Seilrolle lediglich eine gewisse Rollreibung, die aber deutlich geringer als die bei einem feststehend angeordneten Führungskörper auf den Seilzug wirkende Gleitreibung ist.

Zur Realisierung einer synchronen Bewegung der Führungsflächen der Seilführung in gleicher Richtung, kann die Seilführung bei dem erfindungsgemäßen Instrument vorteilhaft in Richtung quer zur Mittelachse des Schafts verschiebbar sein. Dementsprechend ist die Seilführung vorzugsweise in dem Schaft als Ganzes derart verschiebbar gelagert, dass sie von ihrer Normalstellung in eine Richtung quer zur Mittelachse des Schafts und in eine hierzu entgegengesetzte Richtung innerhalb des Schafts bewegt werden kann.

Zu diesem Zweck weist die quer zur Mittelachse des Schafts verschiebbare Führung zweckmäßigerweise einen Schlitten auf, welcher in einer innerhalb des Schafts angeordneten Führung verschiebbar geführt ist. An dem Schlitten sind einander gegenüberliegend die Führungsflächen für die zur Steuerung des Instrumentenkopfs verwendeten beiden Seilzüge ausgebildet und gemäß der bevorzugten Ausgestaltung die die Führungsflächen bildenden Seilrollen drehbar gelagert. Bei der Führung für den Schlitten bzw. die Seilführung handelt es sich um eine in Richtung quer zur Mittelachse des Schafts verlaufende Linearführung, in welcher der Schlitten verschiebbar ist, wobei die Führung zweckmäßigerweise eine Bewegung des Schlittens in axialer Richtung des Schafts verhindert.

Neben einer in Richtung quer zur Mittelachse des Schafts verschiebbaren Ausgestaltung der Seilführung kann die Seilführung auch in anderer Art und Weise relativ zu dem Schaft bewegbar sein. Die Art der kinematischen Ausgestaltung der Seilführung richtet sich hierbei zweckmäßigerweise an den Querschnittsabmessungen des Schafts und an dem innerhalb des Innenlumens des Schafts zur Verfügung stehenden freien Bauraums für die Seilführung. In jedem Fall ist aber sicherzustellen, dass die Bewegung der Seilführung eine Bewegungskomponente in Richtung quer zur Mittelachse des Hohlschafts enthält.

So kann die Seilführung in alternativer vorteilhafter Ausgestaltung zu einer in Richtung quer zur Mittelachse des Schafts verschiebbaren Ausgestaltung in Richtung quer zur Mittelachse des Schafts schwenkbar sein. Zweckmäßigerweise ist die Seilführung hierbei um eine in Richtung der Mittelachse des Schafts von der Seilführung beabstandete Schwenkachse schwenkbar, das heißt, die Seilführung kann in axialer Richtung des Schafts proximalseitig oder distalseitig der Seilführung schwenkbar angelenkt sein.

Ferner kann es auch von Vorteil sein, wenn die Seilführung, wie es gemäß einer anderen zweckmäßigen Ausgestaltung vorgesehen ist, um eine zwischen deren Führungsflächen angeordnete Achse schwenkbar ist. Demzufolge kann die Seilführung in radialer Richtung des Schafts zwischen den beiden Führungsflächen des Schafts schwenkbar an dem Schaft angelenkt sein, so dass bei einer Schwenkbewegung der Seilführung eine der Führungsflächen in radialer Richtung des Schafts von der Mittelachse des Schafts wegbewegt wird, während die andere Führungsfläche in gleicher radialer Richtung des Schafts in Richtung der Mittelachse des Schafts bewegt wird.

Gemäß einer weiteren vorteilhaften Ausgestaltung des erfindungsgemäßen Instruments kann die Seilführung auch sowohl in radialer Richtung des Schafts verschiebbar als auch um eine zwischen den Führungsflächen der Seilführung angeordnete Achse schwenkbar sein, so dass die Seilführung einerseits in einer Linearbewegung in Richtung quer zur Mittelachse des Schafts bewegt werden kann, andererseits aber auch um ein zwischen den Führungsflächen der Seilführung angeordnetes Lager geschwenkt werden kann. Hierbei ist gegebenenfalls auch eine Überlagerung der Linear- und der Schwenkbewegung der Seilführung möglich.

Unabhängig davon, in welcher Weise die Seilführung bewegbar ist, ist sinnvollerweise sicherzustellen, dass die beiden Führungsflächen in ausreichendem Maße quer zur Mittelachse des Schafts bewegbar sind. In diesem Zusammenhang ist weiter zweckmäßig vorgesehen, dass an der Außenwandung des Schafts in Bewegungsrichtung der Seilführung einander diametral gegenüberliegend zwei Ausnehmungen zur Aufnahme der Seilführung ausgebildet sind, wodurch der mögliche Bewegungsweg der Seilführungen vergrößert wird.

Nachfolgend ist die Erfindung anhand von in der Zeichnung dargestellten Ausführungsbeispielen näher erläutert. In der Zeichnung zeigt jeweils schematisch vereinfacht und in unterschiedlichen Maßstäben:
- Fig. 1: in perspektivischer Darstellung einen distalen Endabschnitt eines medizinisch-endoskopischen Instruments mit abgewinkeltem Instrumentenkopf,
- Fig. 2: den distalen Endabschnitt nach Fig. 1 mit einem in Längsverlängerung eines Schafts angeordneten Instrumentenkopf in einem Längsschnitt,
- Fig. 3: die Schnittansicht nach Fig. 2 mit abgewinkeltem Instrumentenkopf,
- Fig. 4: in einer Prinzipdarstellung das medizinisch-endoskopisches Instrument nach Fig. 1,
- Fig. 5: in einer Prinzipdarstellung ein medizinisch-endoskopisches Instrument gemäß einer zweiten Ausgestaltung,
- Fig. 6: in einer Prinzipdarstellung ein medizinisch-endoskopisches Instrument gemäß einer dritten Ausgestaltung,
- Fig. 7: in einer Prinzipdarstellung ein medizinisch-endoskopisches Instrument gemäß einer vierten Ausgestaltung,
- Fig. 8: in einer Prinzipdarstellung ein medizinisch-endoskopisches Instrument gemäß einer fünften Ausgestaltung und
- Fig. 9: in einer Prinzipdarstellung ein medizinisch-endoskopisches Instrument gemäß einer sechsten Ausgestaltung.

Bei dem in der Zeichnung dargestellten medizinisch-endoskopischen Instrument handelt es sich um eine Zange. Dieses Instrument, welches ein manuell zu betätigendes Instrument oder ein in Verbindung mit einem Operationsroboter verwendetes Instrument sein kann, weist einen länglichen, hohlzylindrisch ausgebildeten Schaft 2 auf, wobei in der Zeichnung aus Gründen der besseren Übersichtlichkeit lediglich das distale Ende des Schafts 2 dargestellt ist. Auch die Steuerungseinrichtungen bzw. Antriebe am proximalen Ende des Schafts 2 sind nicht dargestellt, da diese in bekannter Weise ausgebildet sein können.

Das distale Ende des Schafts 2 wird von einem Endstück 4 gebildet. An das Endstück 4 schließt sich distalseitig ein Doppelgelenk 6 an, über welches ein Instrumentenkopf 8 an dem Schaft 2 um eine von einem Gelenkstift 10 gebildete proximale Schwenkachse und um eine von einem Gelenkstift 12 gebildete distale Schwenkachse schwenkbar angelenkt ist. Der Instrumentenkopf 8 bildet einen Werkzeugträger für ein Maulwerkzeug mit zwei relativ zueinander verschwenkbaren Maulteilen 14 und 16.

Das hülsenförmig ausgebildete Endstück 4 des Schafts 2 weist an seinem distalen Ende zwei Vorsprünge 18 und 20 auf, die in Längsverlängerung des Schafts 2 vorstehen und in Richtung quer zur Längsrichtung des Schafts 2 voneinander beabstandet sind. An den Vorsprüngen 18 und 20 ist jeweils ein quer zur Längsausdehnung des Schafts 2 verlaufendes Durchgangsloch ausgebildet, wobei die Durchgangslöcher der beiden Vorsprünge 18 und 20 miteinander fluchten. Diese Durchgangslöcher dienen zur Aufnahme des Gelenkstifts 10, der die proximale Gelenkachse des Doppelgelenks 6 bildet.

Korrespondierend zu den beiden an den Endstück 4 ausgebildeten Vorsprüngen 18 und 20 sind an dem proximalen Ende des Instrumentenkopfs 8 zwei voneinander beabstandete Vorsprünge 22 und 24 ausgebildet, die sich in proximaler Richtung erstrecken. An den Vorsprüngen 22 und 24 sind quer zur Längsausdehnung des Instrumentenkopfs 8 verlaufende und miteinander fluchtende Durchgangslöcher ausgebildet, durch die der die distale Gelenkachse des Doppelgelenks 6 bildende Gelenkstift 12 geführt ist.

An den die proximale und distale Gelenkachse des Doppelgelenks 6 bildenden Gelenkstifte 10 und 12 ist außenseitig der Vorsprünge 18 und 22 ein Gelenkteil 26 angelenkt. In ähnlicher Weise ist an den Gelenkstiften 10 und 12 außenseitig der Vorsprünge 20 und 24 ein weiteres Gelenkteil 28 angelenkt. Die Gelenkteile 26 und 28 bilden einen Teil des Doppelgelenks 6 und verbinden des Instrumentenkopf 8 schwenkbeweglich mit dem Schaft 2. Der Instrumentenkopf 8 ist somit relativ zu dem Schaft 2 in einer Ebene normal zu den Mittelachsen der Gelenkstifte 10 und 12 abwinkelbar, wobei sich seine Abwinkelung aus der Summe der Abwinkelung der Gelenkteile 26 und 28 relativ zu dem Schaft 2 und der Abwinkelung des Instrumentenkopfs 8 relativ zu dem Gelenkteilen 26 und 28 ergibt.

Um eine definierte Abwinkelung des Instrumentenkopfes 8 relativ zu dem Schaft 2 zu ermöglichen, sind das Endstück 4 des Schafts 2 und der Instrumentenkopf 8 über Wälzkörperpaarungen miteinander verbunden. Zur Bildung dieser Wälzkörperpaarungen weisen die distalen Enden der an dem Endstück 4 ausgebildeten Vorsprünge 18 und 20 und die proximalen Enden der an dem Instrumentenkopf 8 ausgebildeten Vorsprünge 22 und 24 jeweils einen Verzahnabschnitt in Form eines Zahnradsegments auf, wobei die an den Vorsprüngen 18 und 20 ausgebildeten Verzahnabschnitte und die an den Vorsprüngen 22 und 24 ausgebildeten Verzahnabschnitte, welche einen identischen Teilkreisdurchmesser aufweisen, miteinander in Eingriff sind.

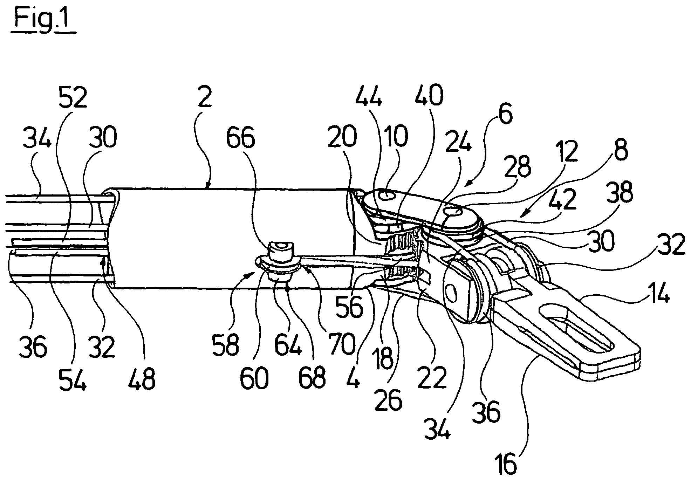

Wie aus Fig. 1 hervorgeht, sind an dem Maulteil 14 zwei Seilzüge 30 und 32 und an dem Maulteil 16 zwei Seilzüge 34 und 36 jeweils antagonistisch wirkend festgelegt. Die Seilzüge 30, 32, 34 und 36 dienen zur Bewegungssteuerung des Maulwerkzeugs und sind über den Bereich des Doppelgelenks 6 durch das Innenlumen des Schafts 2 nach proximalseitig des Schafts 2 geführt und dort mit einer Steuerungseinrichtung wirkungsverbunden.

Jeder der vier zur Bewegungssteuerung des Maulwerkzeugs eingesetzten Seilzüge 30, 32, 34 und 36 wird im Bereich des Doppelgelenks 6 an einem ihm zugeordneten Umlenkrollenpaar umgelenkt. Eines dieser Umlenkrollenpaare, an dem der Seilzug 30 umgelenkt wird, wird von zwei Umlenkrollen 38 und 40 gebildet, wobei die Umlenkrolle 38 außenseitig des Vorsprungs 24 des Instrumentenkopfs 8 auf den Gelenkstift 12 drehbar gelagert ist und die proximalseitig der Umlenkrolle 38 angeordnete Umlenkrolle 40 außenseitig des an dem Endstück 4 des Schafts 2 ausgebildeten Vorsprung 20 drehbar auf dem Gelenkstift 10 gelagert ist. Außenseitig der Umlenkrolle 38 ist auf dem Gelenkstift 12 eine weitere Umlenkrolle 42 drehbar gelagert. Dieser Umlenkrolle 42 bildet zusammen mit einer außenseitig der Umlenkrolle 40 drehbar auf dem Gelenkstift 10 gelagerten Umlenkrolle 44 ein weiteres Umlenkrollenpaar für den Seilzug 34. Aus der Zeichnung nicht ersichtlich, sind korrespondierend zu dem von den Umlenkrollen 38 und 40 gebildeten Umlenkrollenpaar und dem von den Umlenkrollen 42 und 44 gebildeten Umlenkrollenpaar außenseitig des Vorsprungs 22 des Instrumentenkopfs 8 und des Vorsprungs 18 des Endstücks 4 des Schafts 2 weitere Umlenkrollen zur Umlenkung der Seilzüge 32 und 36 angeordnet.

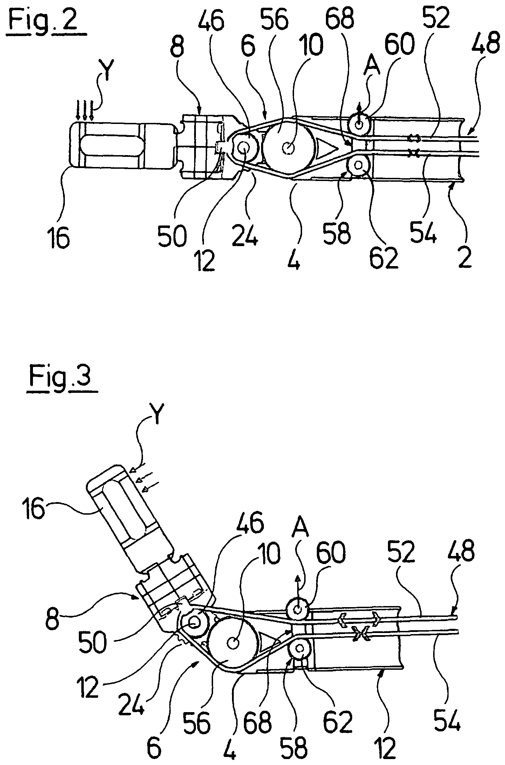

An dem Instrumentenkopf 8 ist in dem Zwischenraum zwischen den an dem Instrumentenkopf 8 ausgebildeten proximalen Vorsprüngen 22 und 24 eine Betätigungsscheibe 46 mit einer kreisförmigen Umfangskontur angeordnet (siehe insbesondere Fig. 2 und 3). Die Betätigungsscheibe 46 ist drehbar auf dem Gelenkstift 12 gelagert und drehfest mit dem Instrumentenkopf 8 verbunden.

Die Betätigungsscheibe 46 wird distalseitig von einem Seil 48 umschlungen, wobei ein mit dem Seil 48 verbundener Zapfen 50 in eine an der Betätigungsscheibe 46 ausgebildete Ausnehmung eingreift, wodurch das Seil 48 formschlüssig an der Betätigungsscheibe 46 festgelegt ist. Die freien Enden des Seils 48 bilden Seilzüge 52 und 54, welche zur Steuerung der Abwinkelung des Instrumentenkopfs 8 relativ zu dem Schaft 2 dienen. Hierzu sind die Seilzüge 52 und 54 durch den Schaft 2 nach proximalseitig des Schafts 2 geführt, wo sie mit einer Steuerungseinrichtung wirkungsverbunden sind.

Proximalseitig der Betätigungsscheibe 46 ist auf dem Gelenkstift 10 in dem Zwischenraum zwischen den distalen Vorsprüngen 18 und 20 des Endabschnitts 4 des Schafts 2 eine Führungsrolle 56 drehbar gelagert. An der Führungsrolle 56, welche einen größeren Außendurchmesser als die Betätigungsscheibe 46 aufweist, sind die Seilzüge 52 und 54 an zwei voneinander abgewandten Umfangsbereichen anliegend geführt.

In einem Bereich am distalen Ende des Schafts 2 und proximalseitig des Endstücks 4 ist bei allen in der Zeichnung dargestellten Ausführungsbeispielen eine Seilführung 58, 58^{I}, 58^{II}, 58^{III}, 58^{IV}, 59^{V} angeordnet. All diesen Seilführungen 58, 58^{I}, 58^{II}, 58^{III}, 58^{IV}, 59^{V} ist gemeinsam, dass sie zwei in Richtung quer zur Mittelachse X des Schafts 2 voneinander beabstandete Führungsflächen für die Seilzüge 52 und 54 aufweisen, welche von zwei drehbar gelagerten Seilrollen 60 und 62 gebildet werden. Darüber hinaus ist bei allen Seilführungen 58, 58^{I}, 58^{II}, 58^{III}, 58^{IV}, 59^{V} der radiale Abstand voneinander geringer als der Durchmesser der Führungsrolle 56. Die Seilzüge 52 und 54 sind zwischen den Seilrollen 60 und 62 hindurchgeführt, wobei der Seilzug 52 an der der Seilrolle 62 zugewandten Seite der Seilrolle 60 und der Seilzug 54 an der der Seilrolle 60 zugewandten Seite der Seilrolle 62 anliegt.

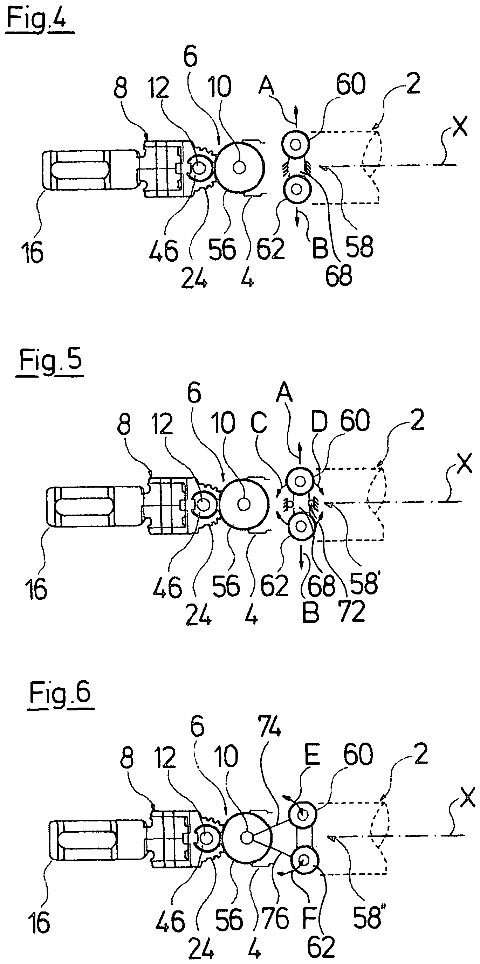

Bei dem in den Fig. 1 - 4 dargestellten Ausführungsbeispiel sind die Seilrollen 60 und 62 mittels zweier Verbindungselemente 64 und 66 miteinander verbunden, welche jeweils außenseitig einer der beiden Flachseiten der Seilrollen 60 und 62 angeordnet sind. Die Verbindungselemente 64 und 66 bilden einen Schlitten 68, der in einer in dem Schaft 2 angeordneten und in Richtungen quer zur Mittelachse X des Schafts 2 ausgerichteten Linearführung, welche in der Zeichnung aus Gründen der besseren Übersichtlichkeit nicht dargestellt ist, in Richtungen A und B (Fig.4) frei verschiebbar ist.

Die Bedeutung der Seilführung 58 wird insbesondere aus den Fig. 2 und 3 deutlich. Wirkt, wie in den Fig. 2 und 3 dargestellt, auf das an dem distalen Ende des Instrumentenkopfs 8 angeordnete Maulwerkzeug eine seitliche Störkraft Y, so hat diese Störkraft Y zur Folge, dass sich die Zugkraft in dem Seilzug 52 vergrößert, wodurch sich auch eine quer zur Mittelachse X des Schafts 2 wirkende Querkraftkomponente der Zugkraft erhöht. Die Erhöhung dieser Querkraftkomponente bewirkt eine Verschiebung der Seilführung 58 in Richtung A, bis sich wieder ein Kräftegleichgewicht zwischen den auf die Seilzüge 52 und 54 wirkenden Seilkräften und der Störkraft Y eingestellt hat, so dass das Maulwerkzeug aufgrund der daran wirkenden Störkraft nicht aus seiner gewünschten Lage relativ zu dem Schaft 2 bewegt wird. Um einen ausreichend großen Bewegungsweg der Seilführung 58 zur Verfügung stellen zu können, sind an dem Schaft 2 an zwei in Bewegungsrichtung der Seilführung 58 diametral gegenüberliegenden Seiten ausgehend von dem distalen Ende des Endabschnitts 4 des Schaftes 2 zwei Ausnehmungen 70 ausgebildet, in welche die Endabschnitte der Seilführung 58 eingreifen können.

Die Seilführung 58^{I} des in Fig. 5 dargestellten Instruments stimmt insoweit mit der Seilführung 58 des in den Fig. 1 - 4 dargestellten Instruments überein, als die Seilrollen 60 und 62 der Seilführung 58^{I} drehbar in einem Schlitten 68 gelagert sind, welcher in einer Linearführung quer zur Mittelachse X des Schafts 2 verschiebbar ist. Die Seilführung 58^{I} unterscheidet sich aber dahingehend von der Seilführung 58, dass der Schlitten 68 um ein zwischen den Seilrollen 60 und 62 angeordnetes Schwenklager 72 schwenkbar ist, so dass die Seilführung 58^{I} aufgrund einer auf das Maulwerkzeug seitlich wirkenden Störkraft Y nicht nur in den Richtungen A und B sondern auch in Richtungen C und D zum Kraftausgleich zwischen den an den Seilzügen 52 und 54 wirkenden Seilkräften und der Störkraft Y schwenkbar ist.

Bei dem in Fig. 6 dargestellten Instrument sind die Seilrollen 60 und 62 der Seilführung 58^{II} um eine von dem Gelenkstift 10 gebildete Achse in Richtung E und hierzu entgegengesetzter Richtung F schwenkbar. Hierbei ist die Seilrolle 60 drehbar an einem proximalen Ende eines an dem Gelenkstift 10 angelenkten Auslegers 74 gelagert und die Seilrolle 62 drehbar an einem proximalen Ende eines an dem Gelenkstift 10 angelenkten Auslegers 76 gelagert.

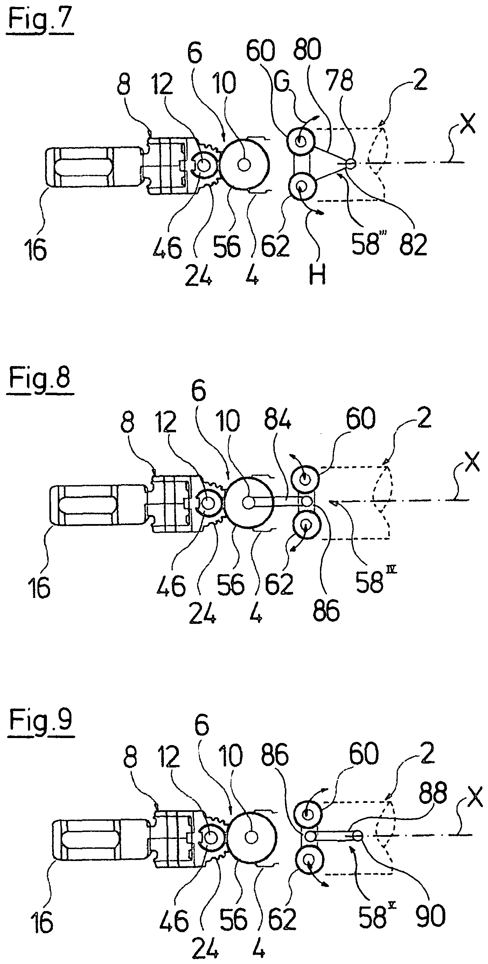

Die Seilrollen 60 und 62 der Seilführung 58^{III} des in Fig. 7 dargestellten Instruments sind gemeinsam um eine proximalseitig der Seilrollen 60 und 62 angeordnete Achse in Richtungen G und H schwenkbar gelagert. Diese Achse wird von einem Stift 78 gebildet, welcher die Mittelachse X des Schafts 2 kreuzt. An dem Stift 78 sind zwei Ausleger 80 und 82 angelenkt, wobei an dem distalen Ende des Auslegers 80 die Seilrolle 60 und an dem distalen Ende des Auslegers 82 die Seilrolle 62 drehbar gelagert ist.

Wie bei dem in Fig. 6 dargestellten Instrument sind die Seilrollen 60 und 62 der Seilführung 58^{IV} des in Fig. 8 dargestellten Instruments um eine von dem Gelenkstift 10 gebildete Achse schwenkbar. Hierzu weist die Seilführung 58^{IV} einen an dem Gelenkstift 10 angelenkten Ausleger 84 auf, an dessen proximalen Ende ein die Seilrollen 60 und 62 miteinander verbindendes Verbindungselement 86 befestigt ist.

Auch bei dem in Fig. 9 dargestellten Instrument sind die Seilrollen 60 und 62 der Seilführung 58^{V} über ein Verbindungselement 86 miteinander verbunden. Das Verbindungselement 86 ist an dem distalen Ende eines Auslegers 88 befestigt, welcher von dem Verbindungselement 86 in proximaler Richtung auskragt und mit seinem proximalen Ende an einem die Mittelachse X des Schafts 2 kreuzend angeordneten Stift 90 angelenkt ist.

### Bezugszeichenliste

- 2: Schaft
- 4: Endstück
- 6: Doppelgelenk
- 8: Instrumentenkopf
- 10: Gelenkstift
- 12: Gelenkstift
- 14: Maulteil
- 16: Maulteil
- 18: Vorsprung
- 20: Vorsprung
- 22: Vorsprung
- 24: Vorsprung
- 26: Gelenkteil
- 28: Gelenkteil
- 30: Seilzug
- 32: Seilzug
- 34: Seilzug
- 36: Seilzug
- 38: Umlenkrolle
- 40: Umlenkrolle
- 42: Umlenkrolle
- 44: Umlenkrolle
- 46: Betätigungsscheibe
- 48: Seil
- 50: Zapfen
- 52: Seilzug
- 54: Seilzug
- 56: Führungsrolle
- 58, 58^{I}, 58^{II}, 58^{III}, 58^{IV}, 59^{V}: Seilführung
- 60: Seilrolle
- 62: Seilrolle
- 64: Verbindungselement
- 66: Verbindungselement
- 68: Schlitten
- 70: Ausnehmung
- 72: Schwenklager
- 74: Ausleger
- 76: Ausleger
- 78: Stift
- 80: Ausleger
- 82: Ausleger
- 84: Ausleger
- 86: Verbindungselement
- 88: Ausleger
- 90: Stift

- A: Richtung
- B: Richtung
- C: Richtung
- D: Richtung
- E: Richtung
- F: Richtung
- G: Richtung
- H: Richtung
- X: Mittelachse
- Y: Störkraft

## Patentansprüche

1. Instrument, insbesondere medizinisch-endoskopisches Instrument, mit einem Schaft (2), mit einem distalseitig des Schafts (2) angeordneten Instrumentenkopf (8), welcher über ein Doppelgelenk (6) mit zwei in Längsrichtung des Instruments voneinander beabstandeten Gelenkachsen an dem Schaft (2) angelenkt ist, und mit zwei durch den Schaft (2) geführten Seilzügen (52, 54), welche mit dem Instrumentenkopf (8) antagonistisch wirkend bewegungsverbunden sind und im Bereich des distalen Endes des Schafts (2) an zwei in radialer Richtung des Schafts (2) einander zugewandten Führungsflächen einer Seilführung (58, 58^{I}, 58^{II}, 58^{III}, 58^{IV}, 59^{V}) anliegen, **dadurch gekennzeichnet, dass** die Führungsflächen der Seilführung (58, 58^{I}, 58^{II}, 58^{III}, 58^{IV}, 59^{V}) synchron in gleicher Richtung quer zu einer Mittelachse (X) des Schafts (2) bewegbar sind.

2. Instrument nach Anspruch 1, **dadurch gekennzeichnet, dass** die Führungsflächen aufgrund einer Kraftwirkung auf einen als Agonist wirkenden Seilzug (52, 54) selbsttätig bewegbar sind.

3. Instrument nach einem der vorangehenden Ansprüche, **dadurch gekennzeichnet, dass** die Seilführung (58, 58^{I}, 58", 58^{III}, 58^{IV}, 59^{V}) zwei in radialer Richtung des Schafts (2) voneinander beabstandete Seilrollen (60, 62) aufweist, welche jeweils eine Führungsfläche für einen Seilzug (52, 54) bilden.

4. Instrument nach einem der vorangehenden Ansprüche, **dadurch gekennzeichnet, dass** die Seilführung (58, 58^{I}) in Richtung quer zur Mittelachse (X) des Schafts (2) verschiebbar ist.

5. Instrument nach Anspruch 4, **dadurch gekennzeichnet, dass** die Seilführung (58, 58^{I}) einen Schlitten (68) aufweist, welcher in einer innerhalb des Schafts (2) angeordneten Führung verschiebbar geführt ist.

6. Instrument nach einem der Ansprüche 1 - 3, **dadurch gekennzeichnet, dass** die Seilführung (58^{II}, 58^{III}, 58^{IV}, 58^{V}) in Richtung quer zur Mittelachse (X) des Schafts (2) schwenkbar ist.

7. Instrument nach Anspruch 6, **dadurch gekennzeichnet, dass** die Seilführung (58^{II}, 58^{III}, 58^{IV}, 58^{V}) um eine in Richtung der Mittelachse (X) des Schafts (2) von der Seilführung (58^{II}, 58^{III}, 58^{IV}, 58^{V}) beabstandete Schwenkachse schwenkbar ist.

8. Instrument nach einem der Ansprüche 1 - 3, **dadurch gekennzeichnet, dass** die Seilführung (58^{I}) um eine zwischen deren Führungsflächen angeordnete Achse schwenkbar ist.

9. Instrument nach einem der vorangehenden Ansprüche, **dadurch gekennzeichnet, dass** die Seilführung (58^{I}) sowohl in radialer Richtung des Schafts (2) verschiebbar als auch um eine zwischen den Führungsflächen der Seilführung (58^{I}) angeordnete Achse schwenkbar ist.

10. Instrument nach einem der vorangehenden Ansprüche, **dadurch gekennzeichnet, dass** an der Außenwandung des Schafts (2) in Bewegungsrichtung der Seilführung einander diametral gegenüberliegend zwei Ausnehmungen (70) zur Aufnahme der Seilführung (58, 58^{I}, 58^{II}, 58^{III}, 58^{IV}, 59^{V}) ausgebildet sind.

## Claims

1. An instrument, in particular a medical-endoscopic instrument, with a shank (2), with an instrument head (8) which is arranged distally of the shank (2) and which is articulated to the shank (2) via a double joint (6) with two joint axes which are distanced to one another in the longitudinal direction of the instrument, and with two pull cables (52, 54) which are led through the shank (2) and which are connected in movement to the instrument head (8) in an antagonistically acting manner and in the region of the distal end of the shank (2) bear on two guide surfaces of a cable guide (58, 58^{I}, 58^{II}, 58^{III}, 58^{IV}, 59^{V}) which face one another in the radial direction of the shank (2), **characterised in that** guide surfaces of the cable guide (58, 58^{I}, 58^{II}, 58^{III}, 58^{IV}, 59^{V}) are movable synchronously in the same direction transversely to a middle axis (X) of the shank (2).

2. An instrument according to claim 1, **characterised in that** the guide surfaces are autonomously movable due to a force action upon a pull cable (52, 54) which acts as an antagonist.

3. An instrument according to one of the preceding claims, **characterised in that** the cable guide (58, 58^{I}, 58", 58^{III}, 58^{IV}, 59^{V}) comprises two cable rollers (60, 62) which are distanced to one another in the radial direction of the shank (2) and which each form a guide surface for a pull cable (52, 54).

4. An instrument according to one of the preceding claims, **characterised in that** the cable guide (58, 58^{I}) is displaceable in a direction transverse to the middle axis (X) of the shank (2).

5. An instrument according to claim 4, **characterised in that** the cable guide (58, 58^{I}) comprises a slide (68) which is displaceably guided in a guide which is arranged within the shank (2).

6. An instrument according to one of the claims 1 - 3, **characterised in that** the cable guide (58^{II}, 58^{III}, 58^{IV}, 58^{V}) is pivotable in the direction transverse to the middle axis (X) of the shaft (2).

7. An instrument according to claim 6, **characterised in that** the cable guide (58^{II}, 58^{III}, 58^{IV}, 58^{V}) is pivotable about a pivot axis which is distanced to the cable guide (58^{II}, 58^{III}, 58^{IV}, 58^{V}) in the direction of the middle axis (X) of the shank (2).

8. An instrument according to one of the claims 1- 3, **characterised in that** the cable guide (58^{I}) is pivotable about an axis which is arranged between its guide surfaces.

9. An instrument according to one of the preceding claims, **characterised in that** the cable guide (58^{I}) is displaceable in the radial direction of the shaft (2) as well as pivotable about an axis which is arranged between the guide surfaces of the cable guide (58^{I}).

10. An instrument according to one of the preceding claims, **characterised in that** two recesses (70) for receiving the cable guide (58, 58^{I}, 58^{II}, 58^{III}, 58^{IV}, 58^{V}) are formed on the outer wall of the shank (2) diametrically opposite one another in the movement direction of the cable guide.

## Revendications

1. Instrument, en particulier instrument d'endoscopie médicale, ayant un manche (2), ayant une tête d'instrument (8) disposée du côté distal du manche (2), qui est articulée sur le manche (2) à l'aide d'une articulation double (6) avec deux axes d'articulation espacés l'un de l'autre dans la direction longitudinale de l'instrument, et ayant deux câbles (52, 54) passés par le manche (2) qui sont reliés en mouvement à la tête d'instrument (8) et agissent de façon antagoniste et qui sont attachés, dans la zone de l'extrémité distale du manche (2), à deux surfaces de guidage, en regard dans la direction radiale du manche (2), d'un guidage de câble (58, 58^{I}, 58^{II}, 58^{III}, 58^{IV}, 58^{V}), **caractérisé en ce que** les surfaces de guidage du guidage de câble (58, 58^{I}, 58^{II}, 58^{III}, 58^{IV}, 58^{V}) sont adaptées pour pouvoir être mues de manière synchrone dans la même direction transversalement à un axe médian (X) du manche (2).

2. Instrument selon la revendication 1, **caractérisé en ce que**, en raison d'un effort exercé sur un câble (52, 54) agissant comme antagoniste, les surfaces de guidage sont susceptibles d'être mues automatiquement.

3. Instrument selon l'une des revendications précédentes, **caractérisé en ce que** le guidage de câble (58, 58^{I}, 58^{II}, 58^{III}, 58^{IV}, 58^{V}) comprend deux galets (60, 62) espacés l'un de l'autre dans une direction radiale du manche (2) qui constituent chacun une surface de guidage pour un câble (52, 54).

4. Instrument selon l'une des revendications précédentes, **caractérisé en ce que** le guidage de câble (58, 58^{I}) est déplaçable par coulissement dans une direction transversale à l'axe médian (X) du manche (2).

5. Instrument selon la revendication 4, **caractérisé en ce que** le guidage de câble (58, 58^{I}) comprend un chariot (68) qui est guidé de manière coulissante dans un guidage disposé à l'intérieur du manche (2).

6. Instrument selon l'une des revendications 1 à 3, **caractérisé en ce que** le guidage de câble (58^{II}, 58^{III}, 58^{IV}, 58^{V}) est adapté pour pouvoir être pivoté dans une direction transversale à l'axe médian (X) du manche (2).

7. Instrument selon la revendication 6, **caractérisé en ce que** le guidage de câble (58^{II}, 58^{III}, 58^{IV}, 58^{V}) est adapté pour pouvoir pivoter autour d'un axe de pivotement espacé du guidage de câble (58^{II}, 58^{III}, 58^{IV}, 58^{V}) en direction de l'axe médian (X) du manche (2).

8. Instrument selon l'une des revendications 1 à 3, **caractérisé en ce que** le guidage de câble (58^{I}) est adapté pour pouvoir pivoter autour d'un axe disposé entre ses surfaces de guidage.

9. Instrument selon l'une des revendications précédentes, **caractérisé en ce que** le câble de guidage (58^{I}) est adapté pour pouvoir être aussi bien déplacé par coulissement dans une direction radiale du manche (2) que pivoté autour d'un axe disposé entre les surfaces de guidage de câble de guidage (58^{I}).

10. Instrument selon l'une des revendications précédentes, **caractérisé en ce que** sont formés sur la paroi extérieure du manche (2), dans la direction de mouvement du câble de guidage diamétralement opposés l'un par rapport à l'autre, deux évidements (70) pour la réception du guidage de câble (58, 58^{I}, 58^{II}, 58^{III}, 58^{IV}, 58^{V}).
